# DEMANDE DE BREVET EUROPEEN

(11) **EP 4 790 412 A1**
(43) Date de publication de la demande: **12.08.2026**
(21) Numéro de dépôt: 26153241.0
(22) Date de dépôt: 21.01.2026
(51) Int. Cl.: G01N 33/24

(54) **DISPOSITIF DE MESURE DE CARACTÉRISTIQUES D'UN SOL ET/OU D'UN SOUS-SOL ET PROCÉDÉ DE MESURE ASSOCIÉ**

(30) Priorité: 07.02.2025 FR 2501273
(71) Demandeur: PURe-Resilience, 75001 Paris (FR)
(72) Inventeur: CASTEL, Franck, 75001 Paris (FR)
(74) Mandataire: Santarelli

(57) **Abrégé**

Dispositif (100, 200) de mesure de propriétés du sol et/ou du sous-sol comprenant :
- un microcontrôleur (10, 20) configuré pour commander l'ensemble des opérations du dispositif ;
- un module de capteurs (15, 25) comprenant au moins une sonde de caractérisation des propriétés du sol et du sous-sol (151, 251), ladite sonde étant configurée pour mesurer au moins deux paramètres physico-chimiques du sol ;
- un module de communication (12, 22) comprenant au moins un composant de communication sans fil, tel qu'un émetteur-récepteur Wi-Fi (122, 222) et/ou Bluetooth (121, 221) ;
- un module de sauvegarde de données (13, 23) configuré pour stocker les mesures collectées ; et
- un module de gestion énergétique (14, 24) comprenant un dispositif de stockage d'énergie (142, 242) ;
le dispositif (100, 200) étant configurable selon au moins trois configurations dîtes de cartographie, d'enregistreur autonome et une combinaison de ces deux configurations.

## Description

### Domaine technique

La présente invention appartient au domaine technique des dispositifs de mesures environnementales.

L'invention concerne plus particulièrement un dispositif de mesure des propriétés du sol et du sous-sol, à la fois portable, autonome et capable d'évaluer les caractéristiques physico-chimiques du sol pouvant être géolocalisées.

L'invention trouve une application directe dans le domaine des appareils de mesure géophysiques, plus particulièrement ceux destinés à l'analyse in situ des sols pour des applications sylvicoles, agricoles, environnementales et industrielles.

### Etat de la technique

Les dispositifs de mesure des propriétés du sol sont depuis longtemps des outils essentiels dans l'agriculture de précision, la gestion des ressources naturelles et les études environnementales. Toutefois, la dégradation progressive des sols, liée à des pratiques agricoles intensives, à l'urbanisation croissante et aux changements climatiques, souligne l'urgence de mieux comprendre l'état de santé des sols et sous-sols.

Cette dégradation se manifeste notamment par une diminution de la fertilité, une perte de biodiversité et une augmentation de l'érosion, compromettant la productivité agricole et les services écosystémiques essentiels.

Une meilleure connaissance des propriétés physico-chimiques du sol permettrait non seulement d'identifier les zones critiques nécessitant une réhabilitation, mais également de développer des stratégies adaptées pour restaurer la qualité des sols.

Par exemple, l'évaluation précise des niveaux de nutriments, de l'humidité ou de la compaction peut guider l'application ciblée d'amendements organiques, tout en évitant les excès nuisibles à l'environnement. Cette connaissance permet également de mettre en place les actions nécessaires au rétablissement d'un éco-équilibre, par une approche de génie pédologique pour reconstituer voire construire des sols fertiles.

En outre, des sols en bonne santé favorisent une biodiversité riche, essentielle à la résistance des écosystèmes face aux perturbations. Il est donc important voire obligatoire de préserver ces écosystèmes qui peuvent, pour certains présenter des fragilités. Le suivi dans le temps de ces réhabilitations est également un élément fondamental pour la réussite de tels projets.

On connaît des dispositifs de mesures ou des capteurs permettant de caractériser des propriétés physico-chimiques des sols et sous-sols. En revanche, ces dispositifs existants présentent quelques inconvénients et ne sont pas particulièrement adaptés à une utilisation in-situ.

Tout d'abord, certains dispositifs connus sont conçus pour des applications spécifiques et manquent de polyvalence. Par exemple, un capteur mesurant l'humidité et la température ne peut pas évaluer la composition chimique du sol, obligeant les opérateurs à recourir à des appareils multiples pour obtenir une analyse complète.

Ensuite, la portabilité et l'autonomie sont des aspects critiques pour les mesures sur le terrain, mais les dispositifs actuels n'offrent pas ces fonctionnalités cumulées. Les capteurs alimentés par des sources externes, ou non étanches, limitent leur utilisation dans des environnements difficiles et/ou isolés.

Cela représente un frein à une meilleure connaissance de l'état de nos sols et sous-sols, limitant alors la mise en place d'actions contribuant à leur amélioration.

Enfin, des dispositifs actuels ne permettent pas une intégration fluide de la géolocalisation et de la mesure des propriétés du sol, ce qui est un autre frein à l'élaboration de cartographies précises pour des analyses à grande échelle, et ne permet pas de caractériser l'hétérogénéité de certains sites et donc d'agir sur des zones spécifiques.

A titre d'exemple, le document FR3043778A1 décrit un système d'évaluation in situ et en continu du potentiel cultural d'un sol agricole comprenant un dispositif d'estimation des flux hydriques et nitriques, comportant des capteurs. Le système est relié à un serveur à distance pour y transmettre les données brutes mesurées par les capteurs, et pour que les étapes de traitement et d'analyse desdites données y soient réalisées.

Le document WO2009084971A1 décrit un système de mesure de différentes propriétés du sol constitué principalement d'une sonde multifonctions qui peut être placée à proximité de la zone des racines de plantes à analyser.

### Présentation de l'invention

La présente invention vise à pallier tout ou partie des inconvénients présentés précédemment.

A cet effet, la présente invention vise un dispositif de mesure de propriétés du sol et/ ou du sous-sol comprenant :
- un microcontrôleur configuré pour commander l'ensemble des opérations du dispositif ;
- un module de capteurs comprenant au moins une sonde de caractérisation des propriétés du sol et du sous-sol, ladite sonde étant configurée pour mesurer au moins deux paramètres physico-chimiques du sol ;
- un module de communication comprenant au moins un composant de communication sans fil, tel qu'un émetteur-récepteur Wi-Fi et/ou Bluetooth ;
- un module de sauvegarde de données configuré pour stocker les mesures collectées ; et
- un module de gestion énergétique comprenant un dispositif de stockage d'énergie.

Le dispositif de mesure des propriétés du sol et/ou du sous-sol est remarquable en ce que ledit dispositif est configurable selon au moins trois configurations, une première configuration dite de cartographie permettant d'effectuer une pluralité de mesures pour étudier l'hétérogénéité d'une surface d'un site d'intérêt, une deuxième configuration dite d'enregistreur autonome permettant d'effectuer des mesures à intervalles de temps prédéfinis et sur une période donnée, et une troisième configuration qui est la combinaison de la première et de la deuxième configurations ; que les modules du dispositif sont remplaçables par connexion/déconnexion pour passer d'une configuration à une autre ; et que le module de gestion énergétique minimise la consommation énergétique du dispositif, via le microcontrôleur.

De façon avantageuse, le dispositif de mesure de propriétés du sol et/ou du sous-sol comprend en outre un récepteur de système de navigation permettant de géolocaliser des mesures obtenues par ledit dispositif, et comprenant en outre un module d'interface homme-machine permettant de vérifier en temps réel lesdites mesures.

Avantageusement, le dispositif de mesure de propriétés du sol et/ou du sous-sol comprend en outre un module de mise en veille et de réveil dudit dispositif.

Selon une caractéristique particulière de l'invention, le module de mise en veille et de réveil dudit dispositif comprenant une horloge interne.

Selon une autre caractéristique particulière de l'invention, le dispositif de mesure de propriétés du sol et/ou du sous-sol comprend en outre un module de commutation intégrant une pluralité de transistors à effet de champ à grille isolée.

Avantageusement, le module de capteurs est évolutif, et est apte à être équipé de plus de cinq sondes.

De façon avantageuse, le module de gestion énergétique comprenant une unité de recharge solaire.

Un autre objet de l'invention est un procédé de mesure des propriétés d'un sol et/ou d'un sous-sol, mis en œuvre par le dispositif de mesure de propriétés du sol et/ou du sous-sol, et qui comprend les étapes suivantes :
- d'initialisation dudit dispositif ;
- d'acquisition des données par le module de capteurs ;
- de sauvegarde desdites données sur un support de stockage local et/ou un serveur distant.

Avantageusement, le procédé de mesure des propriétés d'un sol et/ou d'un sous-sol comprend en outre une étape de géolocalisation.

De façon avantageuse, l'étape d'acquisition des données et l'étape de sauvegarde desdites données est déclenchée selon un planning préalablement défini.

Les concepts fondamentaux de l'invention venant d'être exposés ci-dessus dans leur forme la plus élémentaire, d'autres détails et caractéristiques ressortiront plus clairement à la lecture de la description qui suit et en regard des dessins annexés.

### Présentation des dessins

Les figures sont données à titre purement illustratif pour une meilleure compréhension de l'invention sans en limiter la portée. Les différents éléments peuvent être représentés de manière schématique et ne sont pas nécessairement à la même échelle. Sur l'ensemble des figures, les éléments identiques ou équivalents portent la même référence numérique.

Il est ainsi illustré en :
[Fig.1] : un dispositif de mesure de propriétés du sol et du sous-sol, selon un premier mode de réalisation de l'invention ;
[Fig.2] : des connexions des différents modules composant le dispositif de mesure de propriétés du sol et du sous-sol, selon le premier mode de réalisation de l'invention ;
[Fig.3] : un dispositif de mesure de propriétés du sol et du sous-sol, selon un deuxième mode de réalisation de l'invention ;
[Fig.4] : des connexions des différents modules composant le dispositif de mesure de propriétés du sol et du sous-sol, selon le deuxième mode de réalisation de l'invention ;
[Fig.5] : les étapes d'un procédé de mesure de propriétés du sol et du sous-sol, selon le premier mode de réalisation de l'invention ; et
[Fig.6] : les étapes d'un procédé de mesure de propriétés du sol et du sous-sol, selon le deuxième mode de réalisation de l'invention.

### Description détaillée de modes de réalisation

Il convient de noter que certains éléments techniques bien connus de l'homme du métier sont ici décrits pour éviter toute insuffisance ou ambiguïté dans la compréhension de la présente invention.

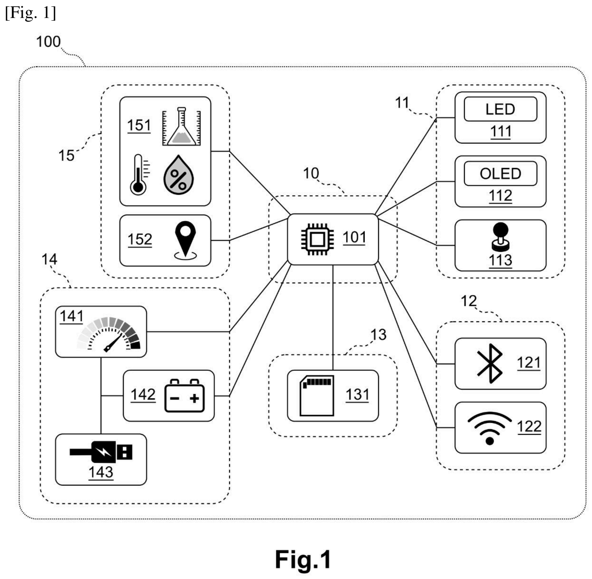

La [Fig.1] représente le schéma d'un dispositif 100 de mesure de propriétés du sol et du sous-sol, selon un premier mode de réalisation de l'invention, ledit dispositif comprenant principalement un microcontrôleur 10, un module d'interface homme-machine 11, un module de communication 12, un module de sauvegarde de données 13, un module de gestion énergétique 14 et un module de capteurs 15.

Avantageusement, l'aspect modulaire du dispositif 100 de mesure de propriétés du sol et du sous-sol permet d'adapter ledit dispositif aux différents besoins de caractérisation, de contribuer à un développement participatif dudit dispositif, en comparaison avec les dispositifs connus de l'art antérieur.

Le microcontrôleur 10 gère les fonctions principales du dispositif 100 de mesure de propriétés du sol et du sous-sol, au moyen d'une puce électronique 101. Notamment, la puce électronique 101 orchestre et commande l'exécution des algorithmes de traitement des données collectées et la coordination entre les différents modules et leur bon fonctionnement.

Associé au microcontrôleur 10, le module d'interface homme-machine 11 est prévu pour permettre à l'opérateur de contrôler et de visualiser des données du dispositif 100. Le module d'interface homme-machine 11 comprend, dans le mode de réalisation préféré, un écran LCD 111 et un écran OLED 112. L'écran LCD 111 et l'écran OLED 112 permettent respectivement de présenter les résultats des mesures et le niveau de tension d'une batterie. Le module d'interface homme-machine 11 comporte en outre un élément de contrôle 113, tel qu'un joystick, une manette, un clavier ou un bouton rotatif, pour permettre à un opérateur d'interagir avec le dispositif 100 de mesure de propriétés du sol et du sous-sol.

Dans un mode de réalisation particulier de l'invention, le module d'interface homme-machine 11 est configuré pour afficher non seulement les données brutes, mais également des représentations graphiques ou des analyses prétraitées. Cela permet une compréhension immédiate des résultats par l'utilisateur.

Le module de communication 12 comporte un émetteur-récepteur Wi-Fi 122 permettant une transmission des données à des plateformes distantes.

Dans un mode de réalisation particulier de l'invention, le module de communication 12 comporte également un émetteur-récepteur Bluetooth 121 pour la connexion sans-fil avec des appareils externes.

Cette architecture de communication offre une grande flexibilité, permettant la consultation des résultats en temps réel ainsi que la synchronisation automatique avec des bases de données distantes.

Dans un mode de réalisation particulier, le module de communication 12 inclut un protocole de sécurisation des données pour garantir la confidentialité et l'intégrité des informations transmises. Ainsi, le traitement supplémentaire des données sur des serveurs cloud est optimisé tout en préservant leur sécurité.

Le module de sauvegarde de données 13 comprend, dans le mode de réalisation préféré de l'invention, un support de mémoire amovible 131, tel qu'une carte SD ou une clé USB, facilitant l'extraction et la conservation des résultats des mesures. Dans un mode de réalisation alternatif, le module de sauvegarde de données 13 comporte un support de mémoire non amovible, non représenté ici, tel qu'une mémoire flash intégrée ou un disque SSD embarqué, offrant une solution robuste pour un stockage sécurisé des données sur le long terme.

Le module de gestion énergétique 14 comporte un contrôleur de tension 141 pour surveiller l'état de la batterie, un dispositif de stockage d'énergie 142 sous forme d'une batterie rechargeable, et un chargeur d'énergie 143. Ces éléments collaborent ensemble pour assurer une autonomie prolongée du dispositif 100 de mesure de propriétés du sol et du sous-sol, ce qui est indispensable pour effectuer des mesures prolongées sur le terrain, notamment dans des environnements isolés ou difficiles d'accès.

Dans un mode de réalisation particulier de l'invention, le module de gestion énergétique 14 comprend en outre un système avancé de gestion énergétique pour optimiser la durée de vie de la batterie en adaptant les cycles de charge basé sur la mise en veille et les réveils conditionnels du microcontrôleur 10, sans nécessiter une gestion active des cycles de charge. Ainsi, le microcontrôleur 10 entre en veille prolongée lorsque les mesures ne sont pas nécessaires, ne se réveille qu'à intervalles définis ou sur interruption externe par l'opérateur via l'élément de contrôle 113 du module d'interface homme-machine 11. De plus, la consommation énergétique des périphériques est réduite, en désactivant ou en mettant en veille les modules du dispositif 100 de mesure de propriétés du sol et du sous-sol qui ne sont pas utilisés entre deux cycles d'acquisition.

Dans un mode de réalisation alternatif, le module de gestion énergétique 14 comprend au moins une capacité de recharge solaire ou à base d'autres sources d'énergies renouvelables, offrant une solution adaptée pour des opérations dans des zones dépourvues d'alimentation électrique.

Le module de capteurs 15 comprend au moins une sonde de caractérisation des propriétés du sol et du sous-sol 151. La sonde 151 est conçue pour mesurer plusieurs paramètres essentiels, notamment l'humidité, la température, le pH, la conductivité électrique et la teneur en nutriments tels que l'azote, le phosphore et le potassium. Ces mesures permettent une évaluation précise des caractéristiques physico-chimiques du sol et d'effectuer un suivi pour diagnostiquer leur état.

Dans un mode de réalisation particulier de l'invention, le module de capteurs 15 comprend un capteur de pression pour mesurer la compaction du sol, qui est un indicateur clé de sa santé structurelle.

Dans d'autres modes de réalisation alternatif de l'invention, le module de capteurs 15 comprend un capteur spectrométrique pour analyser les propriétés optiques du sol, afin d'évaluer la teneur en matière organique ou les contaminants présents. Ces technologies complémentaires renforcent l'utilité du dispositif 100 de mesure de propriétés du sol et du sous-sol pour la réalisation de diagnostics environnementaux.

En outre, le module de capteurs 15 comprend un récepteur de système de navigation 152, tel qu'un GPS, pour enregistrer avec précision la localisation des points de mesure.

Le dispositif 100 de mesure de propriétés du sol et du sous-sol est donc initialement conçu pour effectuer des cartographies de sites d'intérêt.

Dans un mode de réalisation particulier de l'invention, le récepteur de système de navigation 152 intègre des fonctions avancées, telles que la correction différentielle pour améliorer la précision des données géolocalisées.

Dans un mode de réalisation particulier de l'invention, le dispositif 100 de mesure de propriétés du sol et du sous-sol comprend en outre une pluralité de convertisseurs analogiques numériques externes de type ADS115 afin d'être en capacité de connecter plusieurs sondes 151, par exemple jusqu'à 128. Cette configuration particulière, en allouant une adresse unique à chaque sonde et en gérant les délais des réponses des différents signaux de mesure provenant des sondes 151, permet notamment de réaliser plus aisément la cartographie de sites, comme par exemple un carré d'une surface d'environ de 120 m² si les sondes 151 sont séparées d'une distance de 1 m.

Avantageusement, le dispositif 100 de mesure de propriétés du sol et du sous-sol s'utilise également lorsque des forages d'exploration pour prélever un échantillon du sous-sol terrestre sont réalisés (l'échantillon obtenu durant ce type de forage est communément appelé « carotte »).

Ainsi, et directement sur le site d'intérêt, le dispositif 100 de mesure de propriété du sol et du sous-sol est en capacité d'effectuer les mesures au moyen de la sonde 151, ou de la pluralité de sonde 151, de caractérisation des propriétés du sol et du sous-sol. Il devient alors aisé de connaître le gradient des propriétés du sous-sol sur plusieurs mètres de profondeur, lesdites propriétés étant géolocalisées, et pour un site souhaité. A la fin des mesures, la carotte est réinsérée dans le trou de forage, limitant l'impact des campagnes de mesures et ne nécessitant donc pas le transport desdites carottes vers un laboratoire.

Pour garantir une intégration optimale dans des conditions de terrain, le dispositif 100 de mesure de propriétés du sol et du sous-sol et le module capteur 15 sont conçus pour fonctionner préférablement dans une plage, non limitante, de températures de -20°C à 50°C et ledit dispositif ainsi que ledit module sont préférablement étanchéifiés selon l'indice de protection IP68, assurant une résistance aux environnements difficiles présentant des poussières ou exposés à de l'eau (pluie, rosée, arrosage, etc.). Cette conception robuste du dispositif 100 de mesure de propriétés du sol le rend apte à une utilisation fiable dans divers contextes tels que sylvicoles, agricoles, industriels ou environnementaux.

Le dispositif 100 de mesure de propriétés du sol et du sous-sol est alors conçu pour mesurer les propriétés du sol et du sous-sol en associant chaque mesure à une position géolocalisée grâce au récepteur de système de navigation 152. L'opérateur peut déplacer le dispositif 100 de mesure de propriétés du sol et du sous-sol d'un point de mesure à un autre, insérer la sonde de caractérisation des propriétés du sol 151 dans le sol et déclencher la mesure via élément de contrôle 113 intégré. Les données collectées sont sauvegardées sur le support de mémoire amovible 131. Préférablement, et afin de présenter les résultats des mesures sous forme de cartographie de données, les mesures sont prises à un intervalle supérieur ou égal à 50 cm, sans que cela ne présente une limite à la présente invention, cet intervalle étant fonction de la précision du récepteur de système de navigation 152. Cet intervalle permet ensuite d'obtenir une résolution suffisante pour la réalisation des cartographies. Par ailleurs, des ajustements des données collectées par le récepteur de système de navigation 152 peuvent être effectués en post-traitement pour compenser les dérives notamment liées à des facteurs environnementaux, comme la couverture nuageuse ou la proximité de bâtiments qui faussent les mesures de localisation.

Les données sauvegardées sont ensuite récupérées au moyen de plusieurs méthodes : par extraction physique du support de mémoire amovible 131, par téléversement via l'émetteur-récepteur Wi-Fi 122, par téléversement via l' émetteur-récepteur Bluetooth 121 (si présent dans le dispositif 100) ou par téléversement via un câble USB.

Ainsi, cette combinaison de capteurs garantit une analyse exhaustive des propriétés du sol et leur corrélation directe avec les coordonnées géographiques collectées.

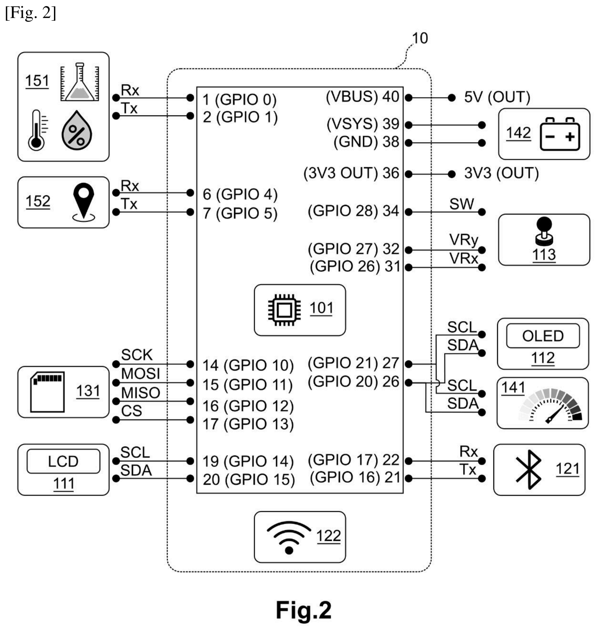

La [Fig.2] représente un schéma d'une architecture interne et des connexions des différents modules du dispositif 100 de mesure de propriétés du sol et du sous-sol, selon un mode de réalisation de l'invention.

La [Fig.2] met en évidence les interconnexions des éléments électroniques et les chemins des signaux entre les différents composants, pour le mode de réalisation préféré de l'invention, dans lequel un microcontrôleur de type *Raspberry Pi Pico W* est utilisé.

Le microcontrôleur 10 constitue l'élément principal de gestion et assure la coordination entre les différents modules du dispositif 100 de mesure de propriétés du sol et du sous-sol. Les connexions aux différents composants sont effectuées au moyen de ports GPIO (en anglais *General Purpose Input*/*Output,* traduit littéralement par *Entrée-sortie à Usage Général*) pour optimiser les flux d'information.

Dans le cas particulier de l'utilisation d'un microcontrôleur de type *Raspberry Pi Pico W*, qui ne présente pas une limite à la présente invention, le tableau 1 ci-dessous résume une configuration optimale des connexions des modules au microcontrôleur 10.

**[Tab.01]**

| élément des différents modules | | port |
|---|---|---|
| nom | connexion | |
| sonde de caractérisation des propriétés du sol 151 | Rx | *GPIO 0* |
| | Tx | *GPIO 1* |
| récepteur de système de navigation 152 | Rx | *GPIO 4* |
| | Tx | *GPIO 5* |
| support de mémoire amovible 131 | SCK | *GPIO 10* |
| | MOSI | *GPIO 11* |
| | MISO | *GPIO 12* |
| | CS | *GPIO 13* |
| écran LCD 111 | SCL | *GPIO 14* |
| | SDA | *GPIO 15* |
| émetteur-récepteur Bluetooth 121 | Rx | *GPIO 16* |
| | Tx | *GPIO 17* |
| contrôleur de tension 141 | SCL | *GPIO 20* |
| | SDA | *GPIO 21* |
| écran OLED 112 | SCL | *GPIO 20* |
| | SDA | *GPIO 21* |
| élément de contrôle 113 | VRx | *GPIO 26* |
| | VRy | *GPIO 27* |
| | SW | *GPIO 28* |
| vers convertisseurs de tension | 3V3 | 3V3 OUT |
| vers convertisseurs de tension | 5V | VBUS |
| dispositif de stockage d'énergie 142 | + | VSYS |
| | - | GND |

La configuration présentée dans le tableau 1 n'est pas un aspect limitatif de la présente invention, et s'adapte à d'autres types de microcontrôleur 10.

Notamment, l'émetteur-récepteur Wi-Fi 122 est ici directement intégré au microcontrôleur 10.

L'optimisation du fonctionnement du dispositif 100 de mesure de propriétés du sol et du sous-sol nécessite un ajustement particulier des tensions d'alimentation des différents modules dudit dispositif. Pour cela, des convertisseurs de tension (non représentés à la [Fig.1] et à la [Fig.2]) sont utilisés entre les modules à alimenter, et les broches d'alimentation *VBUS* et *3V3 OUT.*

Par ailleurs, tous les protocoles de communication ainsi que les ports ne peuvent pas être ouverts en même temps. Le dispositif de mesure de propriétés du sol et du sous-sol fonctionne alors de manière séquentielle pour éviter les conflits internes.

Grâce à la conception modulaire du dispositif 100 de mesure de propriétés du sol et du sous-sol, chaque module s'intègre de manière simplifiée, facilitant ainsi les interconnexions. Le câblage représenté sur la [Fig.2] et résumé dans le tableau 1 minimise les risques d'interférences. Cette approche s'inscrit pleinement dans une démarche privilégiant une solution rationnelle et robuste, et permettant d'optimiser les ressources du microcontrôleur 10 tout en garantissant la durabilité de l'ensemble du dispositif 100 de mesure de propriétés du sol et du sous-sol.

Avantageusement, une optimisation de l'utilisation des ports est réalisée pour éviter les potentiels conflits de protocoles, garantissant une gestion fluide des informations au sein du microcontrôleur 10.

Avantageusement, les ports *GPIO* 20 et *GPIO 21* gèrent simultanément le module OLED 112 pour l'affichage et contrôleur de tension 141, grâce à un partage efficace des ressources *I2C* (en anglais *Inter-Integrated Circuit*, traduit littéralement par Circuit Inter-Intégré), permettant l'ajout optionnel de l'émetteur-récepteur Bluetooth 121, ici sur les ports *GPIO 16* et *GPIO 17* par exemple.

Pour la gestion et le stockage des données, le module de sauvegarde 13, via le support de mémoire amovible 131, est connecté au microcontrôleur 10 via une interface *SPI* (en anglais *Serial Peripheral Interface*, littéralement traduit par Interface Périphérique en Série), utilisant les broches *GPIO 10, GPIO 11, GPIO 12* et *GPIO 13* pour les signaux SCK, MOSI, MISO et CS. Avantageusement, cette configuration assure un stockage rapide et fiable des mesures collectées, tout en offrant plusieurs options d'exportation.

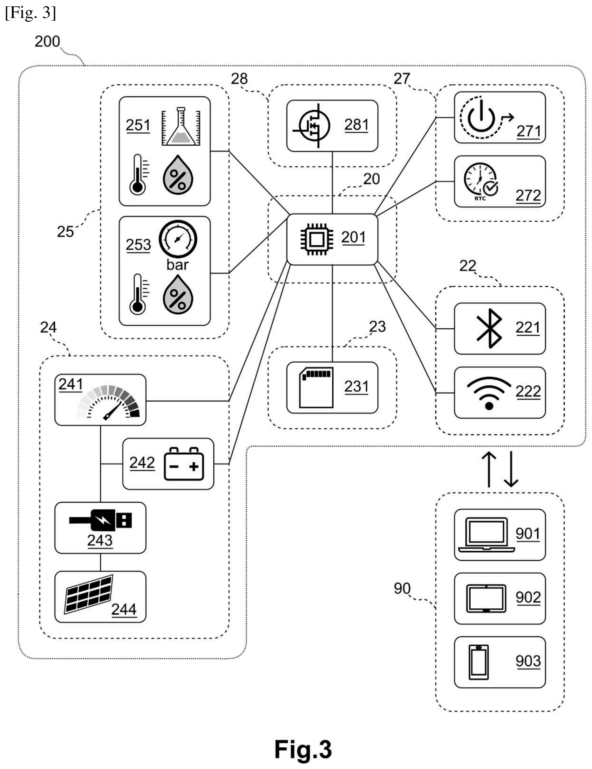

La [Fig.3] représente le schéma d'un dispositif 200 de mesure de propriétés du sol et du sous-sol, selon un deuxième mode de réalisation de l'invention, ledit dispositif comprenant principalement un microcontrôleur 20, un module de communication 22, un module de sauvegarde de données 23, un module de gestion énergétique 24, un module de capteurs 25, un module de mise en/sortie de veille 27 et un module de commutation 28.

Avantageusement, le dispositif 200 de mesure de propriétés du sol et du sous-sol est accessible à distance par au moins un terminal 90 tel qu'un ordinateur 901, via une interface web, une tablette numérique 902 ou un smartphone 903, via des applications mobiles dédiées.

De manière analogue au premier mode de réalisation de l'invention, le microcontrôleur 20 gère les fonctions principales du dispositif 200 de mesure de propriétés du sol et du sous-sol, au moyen d'une puce électronique 201.

Le module de communication 22 comporte, combiné ou non, un émetteur-récepteur Wi-Fi 222 permettant une transmission des données à des plateformes distantes et un émetteur-récepteur Bluetooth 221 pour la connexion sans fil avec des appareils externes.

Il s'agit d'une architecture de communication qui offre une grande flexibilité, pour non seulement permettre la consultation des résultats en temps réel mais également la synchronisation automatique avec des bases de données distantes, en intégrant ou non un protocole de sécurisation des données pour garantir la confidentialité et l'intégrité des informations transmises.

Le module de sauvegarde de données 23 comprend également, et dans le mode de réalisation préféré de l'invention, un support de mémoire amovible 231, tel qu'une carte SD. Le dispositif 200 de mesure de propriétés du sol et du sous-sol ayant pour vocation à être installé sur un site pour des campagnes de mesures en fonction de l'application, de quelques minutes à sur plusieurs mois, le module de sauvegarde de données 23 comporte, dans un mode de réalisation alternatif, un support de mémoire non amovible (non représenté ici), tel qu'une mémoire flash intégrée ou un disque SSD embarqué. Ainsi une courte campagne, dont la durée est de quelques minutes, est par exemple la réalisation d'un diagnostic ponctuel sur un sol agricole ou urbain, une moyenne campagne dont la durée est de quelques jours, est par exemple la réalisation d'un suivi temporel de paramètres d'un sol (humidité, température, conductivité, etc.) pour des projets de végétalisation urbaine ou d'expérimentation agricole, et une longue campagne, dont la durée est de plusieurs mois, est par exemple le suivi temporel des conditions d'un substrat (humidité, température, activité biologique) pour analyser en continu la décomposition et la fertilité dudit substrat.

Comme évoqué, le deuxième mode de réalisation de l'invention a pour vocation à être utilisé pour de longues campagnes de mesure. Ainsi, le module de gestion énergétique 24 intègre plusieurs éléments permettant une autonomie prolongée du dispositif 200 de mesure de propriétés du sol et du sous-sol, et comporte un contrôleur de tension 241 pour surveiller l'état de la batterie, un dispositif de stockage d'énergie 242 sous forme d'une batterie rechargeable, et un chargeur d'énergie 243 et une unité de recharge solaire 244.

Dans un mode de réalisation particulier de l'invention, le module de gestion énergétique 24 comprend en outre un système avancé de gestion énergétique, non représenté ici, pour optimiser la durée de vie de la batterie en adaptant les cycles de charge en fonction notamment de la production d'énergie électrique obtenue par l'unité de recharge solaire 244.

Dans un autre mode de réalisation particulier de l'invention non représenté ici, l'unité de recharge solaire 244 est une unité de recharge fonctionnant à partir d'une autre source d'énergie renouvelable tel que l'éolien ou l'hydrolien.

Le module de capteurs 25 comprend comme précédemment au moins une sonde de caractérisation des propriétés du sol et du sous-sol 251 pour mesurer plusieurs paramètres essentiels tels que l'humidité, la température, le pH, la conductivité électrique ainsi que la teneur en nutriments comme l'azote, le phosphore et le potassium.

Dans un mode de réalisation particulier de l'invention, le dispositif 200 de mesure de propriétés du sol et du sous-sol est apte à acquérir les signaux de mesure de plusieurs sondes 252, par exemple jusqu'à 128, au moyen d'une pluralité de convertisseurs analogiques numériques externes de type ADS115, allouant une adresse unique à chacune desdites sondes et en gérant les délais des réponses des différents signaux de mesure provenant desdites sondes.

Combiné à une optimisation de la consommation énergétique du dispositif 200 de mesure de propriété du sol et du sous-sol, cette configuration présente l'avantage de pouvoir effectuer un suivi dans le temps et sur une surface de terrain conséquente, comme expliqué précédemment. En combinant une pluralité de dispositif 200 de mesure de propriété du sol et du sous-sol, un opérateur est alors en capacité de réaliser plusieurs cartographies sur un site qui est étudié, sur des zones attenantes ou bien des zones localisées à différents endroits d'intérêt sur ledit site.

Dans des modes de réalisation alternatifs, le module de capteurs 25 comprend d'autres types de capteur tel qu'un capteur de pression pour mesurer la compaction du sol, un capteur spectrométrique pour évaluer la teneur en matière organique ou les contaminants présents du sol.

Par ailleurs, le dispositif 200 de mesure de propriétés du sol et du sous-sol est également conçu pour fonctionner préférablement dans une plage, non limitante, de températures de -20°C à 50°C, et ledit dispositif a une étanchéité minimale d'indice de protection IP68.

Dans un mode de réalisation particulier de l'invention, le dispositif 200 de mesure de propriétés du sol et du sous-sol comprend en outre une pluralité de convertisseurs analogiques numériques externes de type ADS115 pour y connecter plusieurs sondes 251, par exemple jusqu'à 128.

Avantageusement, ce mode de réalisation permet de réaliser le suivi temporel de l'état des sols et sous-sols d'une zone prédéfinie.

En outre, le dispositif 200 de mesure de propriétés du sol et du sous-sol comprend une station 253 pour mesurer notamment les variations des conditions atmosphériques de température, de pression et d'humidité.

Cette connaissance des variations temporelles des conditions atmosphériques permet de corriger les mesures réalisées par la sonde 251, les grandeurs mesurées présentant une sensibilité auxdites conditions. Ainsi, le suivi des caractéristiques du sol et du sous-sol sur plusieurs jours est rendu possible au moyen du croisement des données provenant de la sonde 251 et de la station 253.

L'optimisation du fonctionnement du dispositif 200 de mesure de propriétés du sol et du sous-sol est réalisé au moyen du module de mise en/sortie de veille 27 et du module de commutation 28.

Le module de mise en/sortie de veille 27 comprend une interface de sortie de veille 271 et une horloge interne 272.

L'interface de sortie de veille 271 est utilisée pour qu'un opérateur enclenche manuellement au moins une mesure. Dans un mode de réalisation, l'interface de sortie de veille 271 est un élément physique tel qu'un bouton poussoir. Dans un autre mode de réalisation, l'interface de sortie de veille 271 est pilotée à distance, par exemple par Bluetooth. Ces deux modes de réalisation se combinent également.

L'horloge interne 272 est utilisée pour gérer le temps durant une campagne de mesure, et les périodes durant lesquelles le dispositif 200 de mesure de propriétés du sol et du sous-sol est en fonctionnement ou en veille.

Dans le mode de réalisation préféré de l'invention, l'horloge interne 272 est une horloge temps réel (en anglais, *Real-Time Clock* (*RTC*))*.*

L'horloge interne 272 est paramétrable afin de répondre aux besoins des campagnes de mesure tels que la périodicité des mesures, l'autonomie énergétique, la fréquence de transmission des données, la synchronisation de mesures avec d'autres capteurs, la programmation de mesures en fonction de cycles environnementaux.

La gestion de l'alimentation est quant à elle optimisée par le module de commutation 28, qui comprend dans le mode préféré de réalisation de l'invention, une pluralité de transistors à effet de champ à grille isolée, plus couramment nommé MOSFET 281 (acronyme anglais de *Metal -Oxide- Semiconductor Field- Effect Transistor*)*.* Chaque MOSFET 281 est utilisé pour piloter l'alimentation d'un module spécifique du dispositif 200 de mesure de propriétés du sol et du sous-sol, en activant ou en désactivant ledit module de façon indépendante, par rapport aux autres modules, et en fonction de besoins opérationnels qui ont été préalablement définis en amont de la campagne de mesure. Autrement dit, la pluralité de MOSFET 281 joue un rôle essentiel pour réduire la consommation d'énergie du dispositif 200 de mesure de propriétés du sol et du sous-sol, et garantit donc une consommation énergétique adaptée à l'autonomie souhaitée.

Avantageusement, cette configuration contribue à réduire la consommation énergétique du dispositif 200 de mesure de propriétés du sol et du sous-sol, en n'alimentant que le ou les modules nécessaires à un moment donné de la campagne de mesure.

Par ailleurs, ce fonctionnement du dispositif 200 de mesure de propriétés du sol et du sous-sol permet de prolonger la durée de vie du dispositif de stockage d'énergie 242 en fonction de l'utilisation et des périodes d'inactivité dudit dispositif.

Enfin, le module de commutation 28 apporte une flexibilité pour piloter de manière indépendante chaque module du dispositif 200 de mesure de propriétés du sol et du sous-sol, et selon les besoins énergétiques.

Avantageusement, et en fonction de l'énergie restante dans le dispositif de stockage d'énergie 242, le module de commutation 28 peut, via une analyse effectuée par le microcontrôleur 20, enclencher un mode de fonctionnement dit « dégradé » afin de poursuivre la campagne de mesure.

Le mode de fonctionnement dégradé peut par exemple, n'activer qu'une partie du module de capteurs 25, changer les périodes d'activité et d'inactivité en modifiant les consignes au niveau de l'horloge interne 272, ou réduire la fréquence de transmission des données à distance, et cela dans l'attente que le dispositif de stockage d'énergie 242 atteigne à nouveau à une capacité d'autonomie plus élevée.

Le dispositif 200 de mesure de propriétés du sol et du sous-sol est donc initialement conçu pour effectuer des études sur le moyen et le long terme d'un site d'intérêt, en réalisant des mesures à intervalles de temps réguliers ou en réponse à certains évènements tels que le cycle jour/nuit, les variations brutales de température (gel/ dégel, canicule), les chutes de pluie intenses, les périodes de sécheresse ou la détection de secousses (séismes, activités humaines). Ce suivi est possible au moyen de l'optimisation du fonctionnement ainsi que de la consommation énergétique du dispositif 200 de mesure de propriétés du sol et du sous-sol tel que décrit à l'instant.

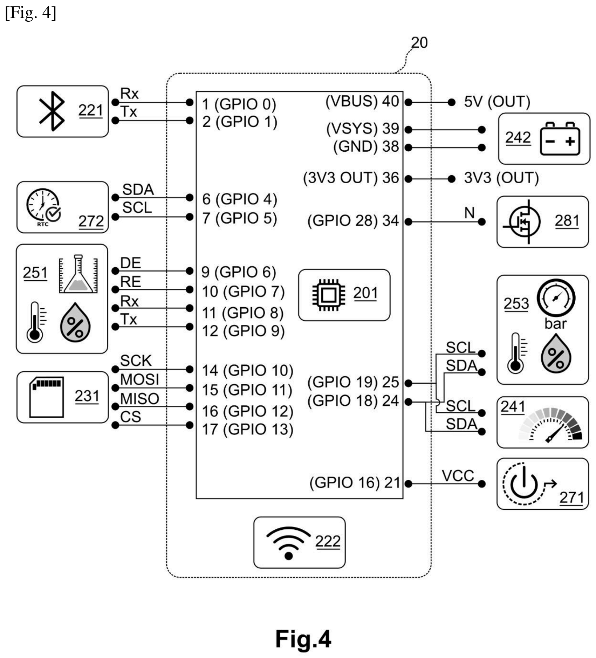

La [Fig.4] représente un schéma d'une architecture interne et des connexions des différents modules du dispositif 200 de mesure de propriétés du sol et du sous-sol, selon le deuxième mode de réalisation de l'invention.

Comme précédemment, la [Fig.4] met en évidence les interconnexions des éléments électroniques et les chemins des signaux entre les différents composants, pour le mode de réalisation préféré de l'invention, dans lequel un microcontrôleur de type *Raspberry Pi Pico W* est utilisé.

Dans le cas particulier de l'utilisation d'un microcontrôleur de type *Raspberry Pi Pico W*, qui ne présente une limite à la présente invention, le tableau 2 ci-dessous résume une configuration optimale des connexions des modules au microcontrôleur 20.

**[Tab.02]**

| élément des différents modules nom | connexion | port |
|---|---|---|
| émetteur-récepteur Bluetooth 221 | Rx | *GPIO 0* |
| | Tx | *GPIO 1* |
| horloge interne 272 | SDA | *GPIO 4* |
| | SCL | *GPIO 5* |
| sonde de caractérisation des propriétés du sol et du sous-sol 251 | DE | *GPIO 6* |
| | RE | *GPIO 7* |
| | Rx | *GPIO 8* |
| | Tx | *GPIO 9* |
| support de mémoire amovible 231 | SCK | *GPIO 10* |
| | MOSI | *GPIO 11* |
| | MISO | *GPIO 12* |
| | CS | *GPIO 13* |
| interface de sortie de veille 271 | VCC | *GPIO 16* |
| contrôleur de tension 241 | SCL | *GPIO 19* |
| | SDA | *GPIO 18* |
| station 253 | SCL | *GPIO 19* |
| | SDA | *GPIO 18* |
| MOSFET 281 | N | *GPIO 28* |
| vers convertisseurs de tension | 3V3 | 3V3 OUT |
| vers convertisseurs de tension | 5V | VBUS |
| dispositif de stockage d'énergie 142 | + | VSYS |
| | - | GND |

Le câblage représenté sur la [Fig.4] et résumé dans le tableau 2 minimise les risques d'interférences, comme évoqué précédemment.

Comme précédemment, une optimisation de l'utilisation des ports est réalisée pour éviter les potentiels conflits de protocoles, garantissant une gestion fluide des informations au sein du microcontrôleur 20.

Avantageusement, le dispositif 200 de mesure de propriétés du sol et du sous-sol offre la possibilité de réaliser des campagnes de mesures in-situ et de caractériser l'état de sols et de sous-sols dans des environnements isolés ou difficiles d'accès offrant ainsi une solution adaptée pour des opérations dans des zones dépourvues d'alimentation électrique. En effet, les dispositifs de l'art antérieur n'ont ni l'autonomie énergétique nécessaire, ni l'optimisation de fonctionnement pour mener de telles campagnes, ou alors qui nécessite de réaliser des prélèvements pour ensuite les faire analyser en laboratoire.

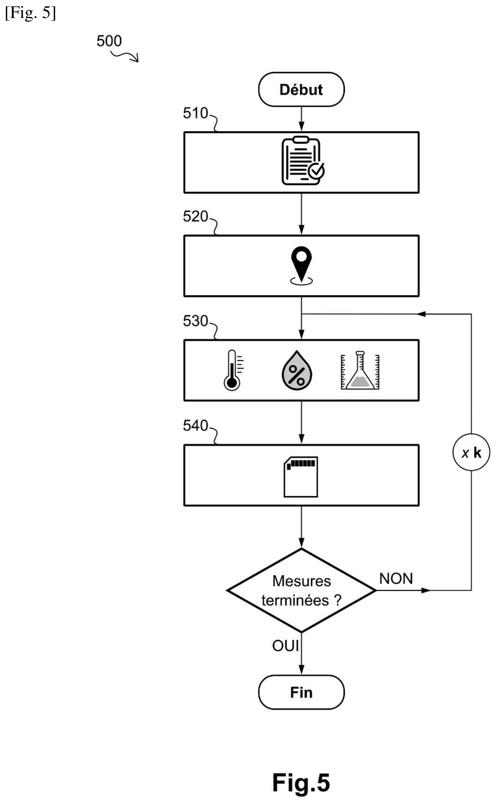

La [Fig.5] représente un schéma d'un procédé de mesure 500 de propriétés du sol et du sous-sol, selon le premier mode de réalisation de l'invention, ledit procédé étant mis en œuvre par le dispositif 100 de mesure de propriétés du sol et du sous-sol.

Le procédé de mesure 500 comprend principalement :
- une étape d'initialisation 510 du dispositif 100 de mesure de propriétés du sol et du sous-sol ;
- une étape de géolocalisation 520 dudit dispositif ;
- une étape d'acquisition 530 de données provenant du module de capteurs 15 dudit dispositif ; et
- une étape de sauvegarde 540 desdites données.

L'étape d'initialisation 510 consiste tout d'abord à mettre sous tension le dispositif 100, puis à vérifier l'état du dispositif de stockage d'énergie 142 et le bon fonctionnement du microcontrôleur 10, du module de capteurs 15 et le module de communication 12 notamment.

L'étape de géolocalisation 520 est ensuite effectuée. L'étape de géolocalisation 520 peut durer plusieurs minutes, notamment cas de couverture nuageuse importante sur le site d'intérêt. La durée de l'étape de géolocalisation 520 à la mise en route du dispositif 100 de mesure de propriétés du sol et du sous-sol présente donc logiquement un certain temps, le temps que les signaux provenant des satellites soient captés par ledit dispositif. Lors des mesures, et si besoin de redéfinir une position du dispositif 100 de mesure de propriétés du sol et du sous-sol, la durée nécessaire pour effectuer cette opération sera plus courte, même en cas d'arrêt dudit dispositif, ou lorsque le signal d'un satellite est perdu en cours de déplacement.

Une fois la géolocalisation effectuée, l'opérateur se rend sur la zone à étudier pour y effectuer des mesures.

Durant l'étape d'acquisition 530 de données, les propriétés du sol et du sous-sol, telles que l'humidité, la température, la conductivité électrique ou d'autres paramètres spécifiques, sont mesurées, l'opérateur ayant pris soin au préalable d'insérer la ou les sondes 151 de caractérisation des propriétés du sol et du sous-sol. Par ailleurs, ces mesures sont affichées en temps réel sur le module d'interface homme-machine 11 du dispositif 100.

Ainsi, et dans un mode de réalisation particulier de l'invention, l'opérateur est apte à valider ou exclure certaines valeurs avant enregistrement.

Une fois les mesures acquises, celles-ci sont enregistrées lors de l'étape 540 de sauvegarde 540 dans le module de stockage de données 13.

L'opérateur peut ensuite réitérer la mesure sur place ou se déplacer à un autre point de mesure, où ledit opérateur pourra vérifier la qualité du signal provenant du récepteur du système de navigation 152.

Avantageusement, le procédé de mesure 500 permet une collecte de données automatisée et optimisée, garantissant la traçabilité et la fiabilité des mesures. De plus, la possibilité d'enregistrer chaque point de mesure avec ses coordonnées de géolocalisation permet d'établir des cartographies précises des propriétés du sol, facilitant ainsi l'analyse comparative et le suivi des évolutions dans le temps.

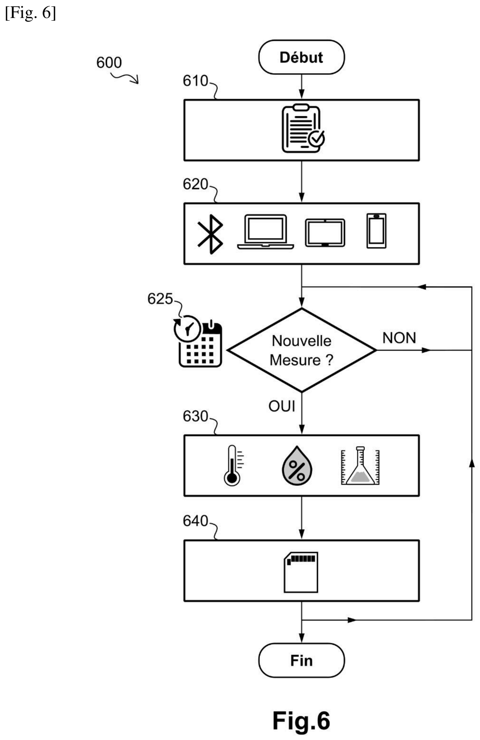

La [Fig.6] représente un schéma d'un procédé de mesure 600 des propriétés du sol et du sous-sol, selon le deuxième mode de réalisation de l'invention, ledit procédé étant mis en œuvre par le dispositif 200 de mesure de propriétés du sol et du sous-sol.

Le procédé de mesure 600 comprend principalement :
- une étape d'initialisation 610 du dispositif 200 de mesure de propriétés du sol et du sous-sol ;
- une étape de connexion 620 avec un terminal distant, permettant à l'opérateur d'interagir avec le dispositif 200 via une interface sans fil ;
- une étape d'acquisition 630 de données provenant du module de capteurs 25 dudit dispositif, et suivant un planning 625 prédéfinis de mesure ; et
- une étape de sauvegarde 640 desdites données.

L'étape d'initialisation 610 consiste tout d'abord à mettre sous tension le dispositif 200, puis à vérifier le bon fonctionnement du microcontrôleur 20, du module de capteurs 25, du module de communication 22 et module de gestion énergétique 24 notamment.

Durant l'étape de connexion 620, une connexion sans fil est établie avec le terminal externe 90 utilisé par l'opérateur, tel qu'un ordinateur, une tablette ou un smartphone, via l'émetteur-récepteur Bluetooth 221 ou l'émetteur-récepteur Wi-Fi 222. Cette connexion permet à l'opérateur de consulter l'état du dispositif 200, de configurer les paramètres dudit dispositif tels que la fréquence d'acquisition des données, les plages de veille et d'activité dudit dispositif en téléversant le planning 625, mais aussi d'exécuter des commandes spécifiques, telles que la synchronisation de l'heure ou la vérification de la batterie.

L'étape de vérification 625 consiste à déterminer si une nouvelle mesure doit être effectuée. Cette décision peut être basée sur un intervalle de temps prédéfini, programmé dans le microcontrôleur 20, ou déclenchée par un événement externe, tel qu'un signal de l'utilisateur ou une condition spécifique.

Si une nouvelle mesure doit être effectuée, le procédé passe à l'étape d'acquisition 630, où les propriétés du sol et du sous-sol sont mesurées par le module de capteurs 25.

Dans un mode de réalisation de l'invention, les mesures sont déclenchées lorsque la différence avec la précédente mesure dépasse un certain seuil. Ainsi, ne sont stockées dans le module de stockage de données 23, que les mesures pertinentes et représentant des variations. Cela permet de limiter l'espace de stockage nécessaire à une campagne de mesure, mais aussi la consommation énergétique nécessaire pour réaliser la sauvegarde des données.

Dans un mode de réalisation particulier de l'invention, les données mesurées peuvent être affichées temporairement sur un terminal connecté via Bluetooth ou Wi-Fi ou équivalent, permettant ainsi à l'opérateur de vérifier ponctuellement leur validité.

Une fois les mesures acquises, celles-ci sont enregistrées lors de l'étape 640 de sauvegarde dans le module de stockage de données 23. Dans un mode de réalisation particulier, les données enregistrées peuvent être automatiquement transférées vers un serveur distant via Wi-Fi ou équivalent, selon une fréquence prédéterminée.

L'étape d'acquisition 630 de données et l'étape de sauvegarde 640 desdites données sont alors déclenchées de nouveau suivant le planning 625 prédéfini de veille/activité.

L'opérateur ou le système détermine alors si une nouvelle mesure doit être effectuée. Si tel est le cas, le procédé retourne à l'étape de vérification 625 pour réitérer le cycle de mesure. Dans le cas contraire (*NON*, voir [Fig.6]), le dispositif passe en mode veille profonde pour préserver l'autonomie de la batterie, ou effectue une clôture sécurisée des données avant son extinction.

Le procédé de mesure 600 permet ainsi une collecte sur une longue durée et qui est optimisée, des propriétés du sol et du sous-sol, particulièrement adaptée aux campagnes de monitoring environnemental, d'agriculture de précision ou d'études écologiques.

## Revendications

1. Dispositif (100, 200) de mesure de propriétés du sol et/ou du sous-sol comprenant :
- un microcontrôleur (10, 20) configuré pour commander l'ensemble des opérations du dispositif ;
- un module de capteurs (15, 25) comprenant au moins une sonde de caractérisation des propriétés du sol et du sous-sol (151, 251), ladite sonde étant configurée pour mesurer au moins deux paramètres physico-chimiques du sol ;
- un module de communication (12, 22) comprenant au moins un composant de communication sans fil, tel qu'un émetteur-récepteur Wi-Fi (122, 222) et/ou Bluetooth (121, 221) ;
- un module de sauvegarde de données (13, 23) configuré pour stocker les mesures collectées ; et
- un module de gestion énergétique (14, 24) comprenant un dispositif de stockage d'énergie (142, 242) ;
**caractérisé en ce que** le dispositif (100, 200) est configurable selon au moins trois configurations, une première configuration dite de cartographie permettant d'effectuer une pluralité de mesures pour étudier l'hétérogénéité d'une surface d'un site d'intérêt, une deuxième configuration dite d'enregistreur autonome permettant d'effectuer des mesures à intervalles de temps prédéfinis et sur une période donnée, et une troisième configuration qui est la combinaison de la première et de la deuxième configurations ; que lesdits modules du dispositif (100, 200) sont remplaçables par connexion/ déconnexion pour passer d'une configuration à une autre ; que le module de gestion énergétique (14, 24) minimise la consommation énergétique du dispositif (100, 200), via le microcontrôleur (10, 20).

2. Dispositif (100) selon la revendication 1, comprenant en outre un récepteur de système de navigation (152) permettant de géolocaliser des mesures obtenues par ledit dispositif, et comprenant en outre un module d'interface homme-machine (11) permettant de vérifier en temps réel lesdites mesures.

3. Dispositif (200) selon l'une quelconque des revendications précédentes, comprenant en outre un module de mise en veille et de réveil (27) dudit dispositif.

4. Dispositif (200) selon la revendication 4, le module de mise en veille et de réveil (27) dudit dispositif comprenant une horloge interne (272).

5. Dispositif (200) selon la revendication 5, ledit dispositif comprenant en outre un module de commutation (28) intégrant une pluralité de transistors à effet de champ à grille isolée (281).

6. Dispositif (100, 200) selon l'une quelconque des revendications précédentes, dans lequel le module de capteurs (15, 25) est évolutif, et est apte à être équipé de plus de cinq sondes (151, 251).

7. Dispositif (100, 200) selon l'une quelconque des revendications précédentes, le module de gestion énergétique (14, 24) comprenant une unité de recharge solaire (244).

8. Procédé (500, 600) de mesure des propriétés d'un sol et/ou d'un sous-sol, mis en œuvre par un dispositif (100, 200) selon l'une quelconque des revendications 1 à 8, comprenant les étapes suivantes :
- d'initialisation (510, 610) dudit dispositif ;
- d'acquisition (530, 630) des données par un module de capteurs (15, 25) ;
- de sauvegarde (540, 640) desdites données sur un support de stockage local (13, 23) et/ou un serveur distant.

9. Procédé (500, 600) selon la revendication 9, comprenant en outre une étape de géolocalisation (520).

10. Procédé (500, 600) selon la revendication 9 ou la revendication 10, l'étape d'acquisition (530, 630) des données et l'étape de sauvegarde (540, 640) desdites données étant déclenchée selon un planning (625) préalablement défini.
